# DEMANDE DE BREVET EUROPEEN

(11) **EP 1 442 736 A1**
(43) Date de publication de la demande: **04.08.2004**
(21) Numéro de dépôt: 03292984.6
(22) Date de dépôt: 01.12.2003
(51) Int. Cl.: A61K 7/48

(54) **Utilisation de l'acide 3-0-acétyl 11-céto-boswellique ou d'un extrait végétal en contenant, pour réduire les rides d'expression**

(30) Priorité: 03.02.2003 FR 0301205
(71) Demandeur: L'OREAL, 75008 Paris (FR)
(72) Inventeur: Meybeck, Alain, 92400 Courbevoie (FR); Zanvit, Alain, 75116 Paris (FR)
(74) Mandataire: Renard, Emmanuelle

(57) **Abrégé**

La présente invention concerne l'utilisation de l'acide 3-O-acétyl 11-céto boswellique ou d'un extrait végétal en contenant, dans une composition adaptée à une application topique sur la peau, comme agent destiné à détendre les traits et/ou décontracter la peau.

Elle concerne également un procédé cosmétique pour détendre les traits et/ou décontracter la peau, comprenant l'application topique sur la peau d'une composition renfermant de l'acide 3-O-acéty 11-céto boswellique ou un extrait végétal en contenant.

L'extrait végétal est en particulier un extrait de *Boswellia serrata*.

## Description

La présente invention concerne l'utilisation de l'acide 3-O-acétyl 11-céto boswellique ou d'un extrait végétal en contenant, dans une composition adaptée à une application topique sur la peau, comme agent destiné à détendre les traits et/ou décontracter la peau.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané ou en prévenant sa dégradation.

Bien que ces traitements permettent d'agir sur les rides et ridules dues au vieillissement chronologique ou intrinsèque, ainsi que de celles dues au photo-vieillissement, ils n'ont pas d'effet sur les rides d'expression.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, les seuls moyens couramment utilisés pour agir sur les rides d'expression sont, d'une part, la toxine botulique qui est notamment injectée dans les rides de la glabelle (voir J.D. Carruters et al., J. Dermatol. Surg. Oncol., 1992, 18, pp. 17-21) et, d'autre part, des implants dégradables à base de collagène, d'acide hyaluronique ou d'acide polylactique.

En outre, comme alternative à ces techniques médicales nécessitant le recours à un praticien, la Demanderesse a proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681 ), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'*Iris pallida* (FR-2 746 641).

Il reste toutefois le besoin de disposer de composés efficaces pour lisser ou estomper les rides d'expression en décontractant la peau ou en décrispant les muscles faciaux.

Or, la Demanderesse a découvert avec étonnement que l'acide 3-O-acétyl 11-céto boswellique permettait de satisfaire ce besoin.

Ce composé est un constituant bien connu de différentes plantes, en particulier du genre Boswellia et plus particulièrement encore de l'espèce *Boswellia serrata*.

*Boswellia serrata* est un arbre d'origine indienne qui fournit un exsudat résineux lorsque son écorce est incisée. Cet exsudat renferme des polysaccharides, des résidus aromatiques et des gommes résiniques, mais surtout des acides terpéniques penta- et tétracycliques, dont l'acide boswellique ainsi que ses dérivés acétylés en position 3 et/ou carbonylés en position 11 (Pardhy & Bhattacharyya, *Ind. J. Chem.,* 16B: 176-178, 1978). Ces composés sont couramment désignés par "acides boswelliques" dans la littérature.

Les acides boswelliques sont connus pour leurs propriétés anti-inflammatoires en raison de leur capacité à inhiber la synthèse des leukotriènes par inhibition de la 5-lipoxygénase [(SINGH G.B. et al, Anti-inflammatory acrions of boswellic acids, *Phytomedicine* 3(1):81-85 (1996), SINGH G.B. et al, Pharmacology of an extract of salai guggal ex-Boswellia serrata, a new non-steroidal anti-inflammatory agent, *Agents and* actions 18(3-4):407-412 (1986), SAFAYHI H. et al, Bowellic acids : novel specific nonredox inhibitors of 5-lipoxygenase, *J. Pharmacol. Exp. Ther.* 261, 1143-1146 (1992)].

Les extraits de *Boswellia serrata* disponibles dans le commerce sont ainsi recommandés contre les irritations cutanées, notamment dans des produits dépilatoires, et l'érythème solaire, en particulier dans des crèmes après-soleil.

D'autres propriétés ont été attribuées aux acides boswelliques et notamment une capacité à inhiber l'activité de l'élastase leucocytaire humaine [SAFAYHI H. et al, Inhibition by boswelic acids of human leukocyte elastase, *J. Pharmacol. Exp. Ther.* 281:460-463 (1997)], ayant conduit à envisager leur utilisation dans le traitement de l'arthrite (EP-0 854 709),. Il a également été démontré qu'ils inhibaient certaines protéases impliquées dans la desquamation cutanée (WO 01/74327) et les collagénases (JP2000-154 131). Il a ainsi été conseillé d'utiliser les extraits de *Boswellia serrata* dans des compositions cosmétiques topiques destinées au traitement des peaux sèches ou âgées, en particulier pour prévenir la formation de rides et ridules.

Toutefois, il n'a encore jamais été suggéré, à la connaissance de la Demanderesse, que les acides boswelliques, et plus particulièrement l'acide 3-O-acétyl 11-céto boswellique, puissent présenter des propriétés justifiant leur utilisation pour décontracter la peau.La présente invention a donc pour objet l'utilisation de l'acide 3-O-acétyl 11-céto boswellique ou d'un extrait végétal en contenant, dans une composition adaptée à une application topique sur la peau, comme agent destiné à détendre les traits et/ou décontracter la peau.

Comme extraits végétaux contenant l'acide 3-O-acétyl 11-céto boswellique, on peut citer les extraits de végétaux du genre Commiphora ou du genre Boswellia, ces derniers étant préférés pour une utilisation dans la présente invention.

Comme extraits végétaux du genre Commiphora, on peut citer les espèces suivantes : *Commiphora myrrha, Commiphora mukul, Commiphora opobalsamum, Commiphora* *playfairii, Commiphora abyssincia* et *Commiphora simplicifolia,* la variété Commiphora *mukul* étant préférée.

Comme extraits végétaux du genre Boswellia, on peut citer les espèces suivantes : *Boswellia serrata, Boswellia carteri, Boswellia papyrifera, Boswellia frereana, Boswellia thurifera, Boswellia glabra, Boswellia oblongata* et *Boswellia socotrana.*

Pour la mise en oeuvre de la présente invention, on préfère utiliser un extrait de *Boswellia serrata*.

Des extraits de *Boswellia serrata* titrés en acide 3-O-acétyl 11-céto boswellique sont disponibles dans le commerce auprès : de la société QUEST sous la dénomination commerciale Soothex®, de la société SABINSA sous la dénomination commerciale Boswellin®, de la société ZANDU sous la dénomination commerciale R3® et de la société NATURACTIVA sous la dénomination commerciale ViaPure Boswellia®.

Ces extraits contiennent entre 0,3 et 3,0% d'acide 3-O-acétyl 11-céto boswellique, par rapport au poids total de l'extrait.

L'extrait végétal utilisé selon l'invention peut en variante être obtenu par extraction d'exsudat résineux de *Boswellia serrata* à l'aide de solvants alcooliques tels que l'éthanol ou l'isopropanol, pour obtenir un extrait alcoolique, éventuellement suivie d'un traitement de l'extrait alcoolique par une solution aqueuse alcaline, destiné à isoler la fraction acide, puis d'une extraction à l'aide d'un solvant organique, tel que l'acétate d'éthyle, pour obtenir une phase aqueuse et une phase organique, et éventuellement d'un traitement acide de l'extrait aqueux obtenu, pour précipiter la fraction acide active de l'extrait.

L'acide 3-O-acétyl 11-céto boswellique peut être isolé à partir de la fraction active précédente par des techniques chromatographiques connues, telles que décrites notamment dans la demande EP-0 755 940, ou encore selon le procédé décrit dans la demande WO 02/085921, ou il peut être obtenu dans le commerce auprès de la société CHROMADEX sous la référence commerciale 02570

La composition selon l'invention est destinée à être appliquée sur les zones du visage ou du front marquées par des rides d'expression, et/ou sur les personnes présentant des rides d'expression.

Elle est ainsi de préférence appliquée sur les sillons nasogéniens, les rides inter-sourcillières et les rides du front. De préférence, la composition est appliquée par massage sur le front.

La quantité d'acide 3-O-acétyl 11-céto boswellique utilisable selon l'invention est bien entendu fonction de l'effet recherché et peut donc varier dans une large mesure. Pour donner un ordre de grandeur, la composition utilisée selon l'invention peut comprendre de 0,001 à 1% en poids, de préférence de 0,001 à 0,1% en poids d'acide 3-O-acétyl 11-céto boswellique, par rapport au poids total de la composition. Si on utilise une source végétale de ce composé, la quantité d'extrait végétal utilisée dans la composition selon l'invention sera ajustée pour que la quantité de dérivé d'acide boswellique corresponde aux pourcentages précités.

La composition selon l'invention est adaptée à une application topique sur la peau et elle contient donc un milieu physiologiquement acceptable, c'est-à-dire compatible avec la peau.

Cette composition peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

La composition selon l'invention peut se présenter sous toutes les formes galéniques normalement utilisées dans le domaine cosmétique, et elle peut être notamment sous forme d'une solution huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale (huile minérale) ou synthétique (huile de vaseline, isohexadécane), les huiles d'origine végétale (huile d'amande d'abricot, fraction liquide de beurre de karité, huile d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'acide 3-O-acétyl 11-céto boswellique.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Comme actifs, il sera avantageux d'introduire dans la composition utilisée selon l'invention au moins un composé choisi parmi : les agents desquamants ; les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ou dermo-décontractants ; les agents tenseurs ; les agents anti-pollution et/ou anti-radicalaire ; et leurs mélanges.

Des exemples de tels composés additionnels sont donnés ci-dessous.

### 1. Agents desquamants

Par "agent desquamant", on entend tout composé capable d'agir :
- soit directement sur la desquamation en favorisant l'exfoliation, tel que les (β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

### 2. Agent hydratant

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA, ses dérivés 7-oxydés et/ou 17-alkylés, les sapogénines et la vitamine D et ses dérivés.

### 3. Agent dépigmentant

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

### 4. Agent anti-glycation

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (*Vaccinium angustifolium)* ; l'ergothionéine et ses dérivés ; et les hydroxystilbènes et leurs dérivés, tels que le resvératrol et le 3,3', 5,5'-tétrahydroxystilbène.

### 5. Inhibiteur de NO-synthase

Des exemples d'inhibiteurs de NO-synthase convenant à une utilisation dans la présente invention comprennent notamment un extrait de végétal de l'espèce *Vitis* *vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

### 6. Agent stimulant la synthèse de macromolécules dermiques ou épidermiques et/ou empêchant leur dégradation

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoffavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelasty® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

### 7. Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl® ; et les lignanes tels que le sécoisolaricirésinol.

### 8. Agent myorelaxant tou dermo-décontractant

Outre le composé de formule (1) décrit précédemment, la composition selon l'invention peut comprendre d'autres agents myorelaxants ou dermo-décontractants, parmi lesquels on peut citer en particulier l'alvérine et ses sels, notamment le citrate d'alvérine, les sapogénines telles que la diosgénine et les extraits naturels en contenant (tels que les extraits de Wild Yam), certaines amines secondaires et tertiaires carbonylées, des sels organiques ou inorganiques de métaux, notamment de manganèse, en particulier le gluconate de manganèse, l'adénosine, ainsi que l'hexapeptide argireline R commercialisé par la société LIPOTEC. On peut également citer certaines compositions parfumantes à effet dermo-décontractant.

### 9. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(3) les silicates mixtes, notamment les phyllosilicates et en particulier les Laponites,
(4) les microparticules de cire, choisies par exemple parmi les cires de Carnauba, de Candelila ou d'Alfa,
(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

### 10. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le coenzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

Selon une forme d'exécution préférée de l'invention, la composition utilisée selon le procédé objet de l'invention renferme, comme actif additionnel, un sel de manganèse, en particulier le gluconate de manganèse.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques plus particulièrement préférés sont choisis parmi les composés suivants (cités selon la nomenclature CTFA ou sous leur dénomination chimique) : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les filtres inorganiques sont de préférence constitués d'oxyde de zinc, de fer, de zirconium, de cérium et/ou de titane (amorphe ou cristallisé sous forme rutile et/ou anatase), de préférence de taille nanométrique (taille moyenne des particules primaires: généralement entre 5 nm et 100 nm, de préférence entre 10 nm et 50 nm), éventuellement enrobés d'alumine et/ou d'acide stéarique.

L'invention sera maintenant illustrée par les exemples non limitatifs suivants.

### EXEMPLES

### Exemple 1 : Mise en évidence de l'effet décontractant d'extraits de Boswellia serrata

On a testé trois extraits de *Boswellia serrata* sur un modèle de co-culture nerfs-muscles qui permet de recréer un arc moteur en innervant des cellules musculaires striées humaines avec des explants de moelle épinière et de ganglions rachidiens d'embryons de rat.

Il s'agissait des extraits commercialisés sous les dénominations commerciales Soothex® par la société QUEST, Boswellin® par la société SABINSA et R3® par la société ZANDU.

Ce test est prédictif d'un effet anti-rides, comme cela a été mis en évidence par la Demanderesse dans le cas du diazépam, qui inhibait les contractions des fibres musculaires dans ce modèle et dont l'activité anti-rides a été démontrée in vivo.

### a) Protocole

Des cellules musculaires humaines, issues de prélèvements de muscle humain de donneur sain, sont ensemencées dans des puits de 15 mm de diamètre (boîtes de culture de 24 puits). Après 10 jours de culture, ces cellules forment une monocouche et fusionnent. A ce stade, des explants de moelle épinière d'embryons de rat de 13 jours contenant le ganglion rachidien sont déposées sur la culture.

Les premières contractions des fibres musculaires sont observées après une semaine de co-culture. Après 3 semaines de co-culture, les fibres musculaires sont striées et possèdent des jonctions neuromusculaires différenciées matures.

Une fibre musculaire ayant des contractions régulières (au moins 60 contractions par minute) est alors sélectionnée dans trois puits de culture différents et le nombre de contractions est comptabilisé sur 30 secondes en utilisant un logiciel d'analyse d'image. Les extraits testés, dilués dans l'éthanol, sont ensuite incubés pendant 60 secondes dans ces puits, aux concentrations C₁ et C₂ de 0,005% et 0,01 %. A la fin de l'incubation, le nombre de contractions est à nouveau comptabilisé sur 30 secondes.

On détermine alors le pourcentage de contractions inhibées. Les résultats sont rassemblés dans le Tableau 1 ci-dessous :

**Tableau 1**

| Effet dermo-décontractant des composés selon l'invention | | |
|---|---|---|
| | **% d'inhibition des contractions** | |
| **Extrait** | **C**_{**1**} **= 0,005%** | **C**_{**2**} **= 0,01%** |
| Soothex® (QUEST) | 26,46% | 37,08% |
| Boswellin® (SABINSA) | 72,14% | -- |
| R3® (ZANDU) | 0,67% | 38,44% |

Ce test démontre ainsi que les extraits de *Boswellia serrata* possèdent un effet myorelaxant ou décontractant qui peut être mis à profit pour la préparation de compositions cosmétiques à effet anti-rides, en particulier pour lisser les rides d'expression.

### Exemple 2 : Mise en évidence de l'effet décontractant de l'acide 3-O-acétyl 11-céto boswellique

Les quatre acides triterpène pentacycliques contenus, selon la littérature, dans l'extrait de *Boswellia serrata*, à savoir l'acide beta-boswellique, l'acide 3-O-acétyl boswellique, l'acide 11-céto boswellique et l'acide 3-O-acétyl 11-céto boswellique, ont été testés dans un modèle de flux calcique pour évaluer leur capacité d'inhibition des canaux calciques et donc leur aptitude à décontracter les traits et lisser les rides d'expression.

Ce test a mis en évidence l'effet décontractant de l'acide 3-O-acétyl 11-céto boswellique, bien supérieur à celui obtenu pour les trois autres acides testés.

Cet effet a été confirmé dans un test identique à celui présenté à l'Exemple 1 qui a montré un effet d'inhibition des contractions de 74,7% à 5 µM et de 87% à 10 µM pour ce composé.

### Exemple 3 : Composition cosmétique

Cette composition est préparée de manière classique pour l'homme du métier. Les quantités données dans cet exemple sont indiquées en pourcentages pondéraux.

| | |
|---|---|
| Extrait de *Boswellia serrata* (Soothex® de QUEST) | 3,00 % |
| Huiles | 18,00 % |
| EDTA disodique | 0,1 % |
| Silicate mixte (Laponite XLG de ROCKWOOD) | 1,0 % |
| Triéthanolamine | 0,5 % |
| Filtres UV | 2,0 % |
| Charges | 4,0 % |
| Glycérine | 7,0 % |
| Acide stéarique | 3,0 % |
| Mélange de stéarate de glycéryle et de stéarate de | |
| polyéthylène glycol (100 OE) | 2,0 % |
| Stéarate de polyéthylène glycol (20 OE) | 0,80 % |
| Alcools gras | 1,0 % |
| Conservateurs | 0,60 % |
| Eau | qsp 100 % |

Cette crème est destinée à être appliquée sur le visage et le front pour décontracter le visage.

## Revendications

1. Utilisation de l'acide 3-O-acétyl 11-céto boswellique ou d'un extrait végétal en contenant, dans une composition adaptée à une application topique sur la peau, comme agent destiné à détendre les traits et/ou décontracter la peau.

2. Utilisation selon la revendication 1, **caractérisée en ce que** l'extrait végétal est choisi parmi les extraits végétaux du genre Boswellia et les extraits végétaux du genre Commiphora.

3. Utilisation selon la revendication 2, **caractérisée en ce que** les extraits végétaux du genre Commiphora sont choisis parmi les espèces suivantes : *Commiphora myrrha, Commiphora mukul, Commiphora opobalsamum, Commiphora playfairii, Commiphora abyssincia* et *Commiphora simplicifolia.*

4. Utilisation selon la revendication 3, **caractérisée en ce que** l'extrait végétal est un extrait de *Commiphora mukul.*

5. Utilisation selon la revendication 2, **caractérisée en ce que** les extraits végétaux du genre Boswellia sont choisis parmi les espèces suivantes : *Boswellia serrata, Boswellia carteri, Boswellia papyrifera, Boswellia frereana, Boswellia thurifera, Boswellia glabra, Boswellia oblongata* et *Boswellia socotrana.*

6. Utilisation selon la revendication 5, **caractérisée en ce que** l'extrait végétal est un extrait de *Boswellia serrata*.

7. Utilisation selon la revendication 6, **caractérisée en ce que** ledit extrait est susceptible d'être obtenu par extraction d'exsudat résineux de *Boswellia serrata* à l'aide de solvants alcooliques tels que l'éthanol ou l'isopropanol pour obtenir un extrait alcoolique.

8. Utilisation selon la revendication 7, **caractérisée en ce que** l'étape d'extraction est suivie d'un traitement de l'extrait alcoolique par une solution aqueuse alcaline.

9. Utilisation selon la revendication 8, **caractérisée en ce que** le traitement alcalin est suivi d'une extraction à l'aide d'un solvant organique, tel que l'acétate d'éthyle, pour obtenir une phase aqueuse et une phase organique.

10. Utilisation selon la revendication 9, **caractérisée en ce que** l'extraction est suivie d'un traitement acide de l'extrait aqueux obtenu.

11. Utilisation selon l'une quelconque des revendications 1 à 10, **caractérisée en ce que** la composition comprend de 0,001 % à 0,1% en poids d'acide 3-O-acétyl 11-céto boswellique, par rapport au poids total de la composition.

12. Utilisation selon l'une quelconque des revendications 1 à 11, **caractérisée en ce que** la composition renferme en outre un sel de manganèse.

13. Utilisation selon la revendication 12, **caractérisé en ce que** le sel de manganèse est le gluconate de manganèse.

14. Utilisation selon l'une quelconque des revendications 1 à 13, **caractérisée en ce que** la composition est appliquée surles sillons nasogéniens, les rides inter-sourcillières et/ou les rides du front.
